# EUROPÄISCHE PATENTSCHRIFT

(11) **EP 1 832 573 B1**
(45) Veröffentlichungstag und Bekanntmachung des Hinweises auf die Patenterteilung: **21.12.2011**
(21) Anmeldenummer: 07004561.2
(22) Anmeldetag: 06.03.2007
(51) Int. Cl.: C07C 275/62, C08G 18/28, C08G 18/32, C08G 18/50, C08G 18/79, C09D 5/04, C09D 7/12, C09D 7/02, C09D 7/00

(54) **Biuretverbindungen zur Rheologiesteuerung**
Buret connections for rheology control
Combinaison de biuret destinée à la commande rhéologique

(30) Priorität: 08.03.2006 DE 102006010721
(43) Veröffentlichungstag der Anmeldung: 12.09.2007
(73) Patentinhaber: BYK-Chemie GmbH, 46483 Wesel (DE)
(72) Erfinder: Haubennestel, Karlheinz, 46487 Wesel (DE); Orth, Ulrich, Dr., 46485 Wesel (DE); Pickavé, Matthias, 45479 Mülheim an der Ruhr (DE); Mößmer, Stefan, Dr., 46499 Hamminkeln (DE); Leutfeld, Daniela, 46485 Wesel (DE)
(74) Vertreter: Leifert & Steffan

(56) Entgegenhaltungen:
- EP-A- 1 048 681
- EP-A- 1 593 700

## Beschreibung

Die Erfindung betrifft Biuretverbindungen, Verfahren zu ihrer Herstellung und ihre Verwendung als Mittel zur Rheologiesteuerung von Beschichtungssystemen wie z. B. lösungsmittelhaltige, lösungsmittelfreie und wässrige Lacke, PVC-Plastisolen, Beschichtungen auf Epoxidbasis und auf Basis ungesättigter Polyesterharze.

Um die Rheologie von flüssigen Beschichtungssystemen zu steuern, werden vielfach Kieselsäuren, hydriertes Rizinusöl oder organisch modifizierte Bentonite, wie z. B. in den US-Patenten 4,208,218; 4,410,364 und 4,412,018 beschrieben, eingesetzt. Desweiteren kommen vielfach Polyamidwachse zum Einsatz. Gerade auf dem Gebiet der Polyamide und Polyamidester existieren zahlreiche Patente, wie z. B. DE 69523221; EP 0528363; EP 0239419; US 5,510,452 und US 5,349,011.
Es werden aber auch Kombinationen von modifizierten Bentoniten mit Polyamiden, wie in der EP 0509202 und DE 69704691 beschrieben, eingesetzt.

Nachteilig bei diesen Stoffen ist, daß sie meist trockene Feststoffe oder Pasten darstellen, die mittels Lösungsmitteln und Scherkräften zu einem Halbfabrikat aufgeschlossen bzw. durch gezielte Temperatursteuerung in das flüssige Beschichtungssystem eingebracht werden müssen. Werden diese Temperaturen nicht eingehalten, treten im fertigen Beschichtungssytem Kristallite auf, die zu Fehlern in der Beschichtung führen. Der generelle Nachteil dieser Systeme ist, daß sie zu Trübungen und Schleierbildungen (Haze) in klaren, transparenten Beschichtungen führen. Außerdem ist der Umgang mit trockenen Produkten, die Stäube bei der Verarbeitung verursachen, nicht gewünscht.
Die Polyamidester sind zwar häufig flüssig und daher deutlich weniger wirksam als die von Natur aus festen Stoffe.

Andere Lösungen zur Rheologiesteuerung wurden dargestellt in der EP 0 198 519. Hier wird ein Isocyanat mit einem Amin in Gegenwart von Bindemitteln zu einem Harnstoff umgesetzt, der in feinst disperser Form nadelförmige Kristalle bildet. Diese so modifizierten Bindemittel werden als rheologiesteuernde und ablaufverhindernde Bindemittel angeboten, als sogenannte "sag control agents".

Der Nachteil dieser Produkte liegt darin begründet, daß sie immer an diese Bindemittel gebunden sind, in denen sie hergestellt wurden und nicht eine nachträgliche universelle Korrektur von fertigen Beschichtungsmitteln zulassen.

In der EP 1 048 681 A wird ein Verfahren zur Herstellung eines Thixotropiemittels offenbart, das durch Umsetzung von Monohydroxyverbindungen mit Diisocyanat und durch weitere Umsetzung mit Diaminen zu Harnstoffurethanen erhalten wird.

In der EP 0 006 252 wird ein Verfahren zur Herstellung eines Thixotropiemittels beschrieben, das einige der o. g. Nachteile ausräumt, in dem es Harnstoffurethane beschreibt, die in aprotischen Lösungsmitteln in Gegenwart von LiCl durch Umsetzung von Isocyanataddukten mit Polyaminen hergestellt werden. Der Nachteil der so hergestellten Produkte liegt in der durch das Herstellverfahren bedingten undefinierten Struktur dieser Harnstoffurethane. Bei diesem Verfahren wird 1 mol eines Diisocyanats zunächst mit 1 mol eines Monoalkohols umgesetzt. Dabei entstehen die gewünschten NCO-funktionellen Monoaddukte, aber auch nicht-NCOfunktionelle Diaddukte. Außerdem bleibt ein gewisser Anteil an monomerem Diisocyanat nicht umgesetzt. Die Anteile dieser verschiedenen Verbindungen können schwanken, je nach Zugänglichkeit der NCO-Gruppe und der angewandten Reaktionsführung, wie Temperatur und Zeit. Alle diese so hergestellten Addukte enthalten jedoch größere Mengen nicht umgesetztes Diisocyanat, welches bei der weiteren Umsetzung mit Polyaminen zu unkontrollierter Kettenverlängerung des Moleküls führt. Diese Produkte neigen dann zu Ausfällungserscheinungen oder vorzeitiger Gelierung und demzufolge zur Bildung von sogenannten "Stippen" ("seeds") im Bindemittel. In der DE 19919482 werden diese Nachteile durch Entfernen des überschüssigen Isocyanates umgangen. Diese Produkte haben jedoch den Nachteil, dass sie nur in hochpolaren Lösemitteln wie z. B. NMP unter Zuhilfenahme von Alkalisalzen stabile Lösungen liefern.

Die vorliegende Erfindung hat sich daher die Aufgabe gestellt, ein Verfahren zu finden, welches Thixotropiemittel einer definierteren Struktur erzeugt und damit ein besseres Wirkungsprofil und eine bessere Reproduzierbarkeit der Thixotropierung gewährleistet.

Überraschenderweise wurde gefunden, daß diese Aufgabenstellung gelöst werden kann durch Biuretverbindungen, die aus Uretdionen (Komponente A) und monoaminfunktionellen Verbindungen (Komponente B) herstellbar sind und die allgemeine Struktur A-B aufweisen.

Gegenstand der Erfindung sind daher Biuretverbindungen der idealisierten allgemeinen Formel 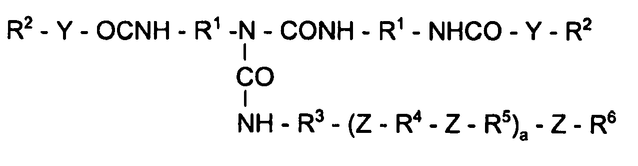 in der
R¹ für einen (C₁-C₂₂)-Alkylen-, (C₃-C₂₂)-Alkenylen-, (C₅-C₁₅)-Cycloalkylen-, Arylen-, (C₇-C₁₂)-Aralkylen-, einen Polyoxyalkylen-Rest oder für einen Polyester-Rest steht,
R² für einen (C₁-C₂₂)-Alkyl-, Hydroxy-(C₁-C₂₂)-Alkyl-, (C₃-C₁₈)-Alkenyl, Aryl-, (C₇-C₁₂)-Aralkyl-, oder (C₅-C₁₂)-Cycloalkyl-Rest, einen Hydroxy-, (C₁-C₂₂)-Alkoxy-, (C₅-C₁₂)-Cycloalkoxy-, oder (C₇-C₁₂)-Aralkoxy-Polyoxyalkylen-Rest, einen (C₁-C₂₂)-Alkanol-, (C₅-C₁₂)-Cycloalkanol-, oder (C₇-C₁₂)-Aralkanol-gestarteten oder einen (C₁-C₂₂)-Alkoxy-, (C₆-C₁₂)-Cycloalkoxy-, oder (C₇-C₁₂)-Aralkoxy-Polyoxyalkylengestarteten Polyester steht,
Y für gleiche oder verschiedene Reste O, NH, CO-NH-NH oder NH-NH-CO steht
R³, R⁴ und R⁵ unabhängig voneinander für einen (C₂-C₄₀)-Alkylen-, (C₃-C₄₀)-Alkenylen-, (C₅-C₄₀)-Cycloalkylen-, Arylen-, (C₇-C₄₀)-Aralkylen- oder Polyoxyalkylen-Rest oder für einen Polyester-Rest stehen,
R⁶ für einen (C₁-C₃₀)-Alkyl-, (C₃-C₂₂)-Alkenyl-, Hydroxyalkyl- und -alkenyl-, (C₄-C₁₃)-Cycloalkyl-, Aryl- oder (C₇-C₁₂)-Aralkyl-Rest steht,
Z für eine oder mehrere der folgenden Gruppen COO, OCO, NHCO, CONH, NHCOO, OOCNH, NHCONH steht und
a für eine Zahl von 1 bis 19 steht.

Die Definitionen der Reste R¹, R², R³, R⁴, R⁵, R⁶, Y und Z sowie des Index a entsprechen im Rahmen dieser Erfindung unabhängig von den Verbindungen, in welchen diese aufgeführt sind, den obigen Definitionen. Bevorzugte Ausführungen dieser Reste finden sich in den jeweiligen Unterabschnitten.

Im Falle, dass einer oder mehrere der Reste R¹, R², R³, R⁴ und/oder R⁵ einen Polyoxyalkylen-Anteil enthalten, sind diese Reste unabhängig von den Verbindungen, in welchen diese aufgeführt sind, vorzugsweise aus Ethylenoxid, Propylenoxid und/oder Butylenoxid-Einheiten, statistisch oder blockweise angeordnet, aufgebaut, und gegebenenfalls ist einer oder sind mehrere dieser Einheiten durch Styrol-Einheiten substituiert. Besonders bevorzugt sind Ethylenoxidund Propylenoxid-Reste.

Im Falle, dass einer oder mehrere der Reste R¹, R², R³, R⁴ und/oder R⁵ einen Polyester-Rest umfassen, sind diese Reste unabhängig von den Verbindungen, in welchen diese aufgeführt sind, vorzugsweise auf der Basis von ein oder mehreren (C₁-C₁₈)-Hydroxycarbonsäuren oder ein oder mehreren Lactonen, wie β-Propiolacton, ö-Valerolacton, ε-Caprolacton und (C₁-C₆)-alkylsubstituiertem ε-Caprolacton, aufgebaut.

R¹ steht besonders bevorzugt für einen Hexamethylenrest.

R² steht vorzugsweise für einen (C₁-C₂₂)-Alkyl- oder einen -Alkoxypolyoxyalkylenrest.

Die Reste R³ und R⁵ stehen unabhängig voneinander vorzugsweise für einen (C₂-C₁₈)-Alkylen-, (C₇-C₁₅)-Aralkylen-Rest, besonders bevorzugt für einen (C₂-C₁₂)-Alkylen-, (C₇-C₁₂)-Aralkylen-Rest, ganz besonders bevorzugt für einen (C₂-C₈)-Alkylen-, (C₇-C₉)-Aralkylen-Rest, wie beispielsweise einen Hexamethylen-, Octamethylen- oder m-Xylylen-Rest. Bevorzugt sind die Reste R³ und R⁵ identisch.

R⁴ steht für einen (C₂-C₄₀)-Alkylen-, (C₃-C₄₀)-Alkenylen-, (C₅-C₄₀)-Cycloalkylen-, Arylen-oder (C₇-C₄₀)-Aralkylen-Rest, bevorzugt für einen (C₃₀-C₄₀)-Alkylen-, (C₃₀-C₄₀)-Alkenylen-, (C₃₀-C₄₀)-Cycloalkylen-, Arylen-, oder (C₃₀-C₄₀)-Aralkylen-Rest, wie beispielsweise den Rest zwischen den beiden Carbonsäuregruppen der Dimersäure. Besonders bevorzugt steht R⁴ für einen C₃₄-Rest.

R⁶ steht vorzugsweise für einen (C₁-C₃₀)-Alkyl- oder einen (C₃-C₂₂)-Alkenyl-Rest, welche eventuell mit Hydroxygruppen substituiert sein können, besonders bevorzugt einen (C₁₂-C₃₀)-Alkyl- oder einen (C₁₂-C₂₂)-Alkenyl-Rest und ganz besonders bevorzugt einen (C₁₂-C₂₀)-Alkyl- oder einen (C₁₂-C₂₀)-Alkenyl-Rest, wie beispielsweise einen C₁₇-Alkyl- oder einen C₁₇-Alkenyl-Rest.

Z steht besonders bevorzugt für NHCO und CONH.

Der Index a steht für eine Zahl von 1 bis 19, besonders bevorzugt von 2 bis 7.
Gegenstand der Erfindung ist auch ein Verfahren zur Herstellung thixotropieerzeugender und ablaufverhindernder Biuretverbindungen der allgemeinen Struktur A-B, erhältlich durch Umsetzung von Uretdionen der idealisierten allgemeinen Formel (A) 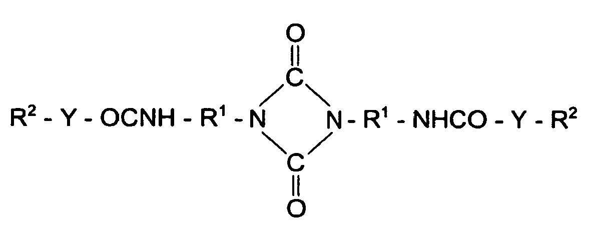 wobei R¹ für einen (C₁-C₂₂)-Alkylen-, (C₃-C₂₂)-Alkenylen-, (C₅-C₁₅)-Cycloalkylen-, Arylen-, (C₇-C₁₂)-Aralkylen-, einen Polyoxyalkylen-Rest oder für einen Polyester-Rest steht,
R² für einen (C₁-C₂₂)-Alkyl-, Hydroxy-(C₁-C₂₂)-Alkyl-, (C₃-C₁₈)-Alkenyl, Aryl-, (C₇-C₁₂)-Aralkyl-, oder (C₅-C₁₂)-Cycloalkyl-Rest, einen Hydroxy-, (C₁-C₂₂)-Alkoxy-, (C₅-C₁₂)-Cycloalkoxy-, oder (C₇-C₁₂)-Aralkoxy-Polyoxyalkylen-Rest, einen (C₁-C₂₂)-Alkanol-, (C₅-C₁₂)-Cycloalkanol-, oder (C₇-C₁₂)-Aralkanol-gestarteten oder einen (C₁-C₂₂)-Alkoxy-, (C₆-C₁₂)-Cy_{d}oalkoxy-, oder (C₇-C₁₂)-Aralkoxy-Polyoxyalkylen-gestarteten Polyester steht,
Y für gleiche oder verschiedene Reste 0, NH, CO-NH-NH oder NH-NH-CO steht,
mit monoaminfunktionellen Verbindungen der idealisierten allgemeinen Struktur (B)

(B) H₂N-R³-[Z-R⁴-Z-R⁵]ₐ-Z-R⁶

wobei R³, R⁴ und R⁵ unabhängig voneinander für einen (C₂-C₄₀)-Alkylen-, (C₃-C₄₀)-Alkenylen-, (C₅-C₄₀)-Cycloalkylen-, Arylen-, (C₇-C₄₀)-Aralkylen- oder Polyoxyalkylen-Rest oder für einen Polyester-Rest stehen,
R⁶ für einen (C₁-C₃₀)-Alkyl-, (C₃-C₂₂)-Alkenyl-, Hydroxyalkyl- und -alkenyl-, (C₄-C₁₃)-Cycloalkyl-, Aryl- oder (C₇-C₁₂)-Aralkyl-Rest steht,
Z für eine oder mehrere der folgenden Gruppen COO, OCO, NHCO, CONH, NHCOO, OOCNH, NHCONH steht und
a für eine Zahl von 1 bis 19 steht.

Bei der Herstellung der erfindungsgemäßen Biuretverbindungen der allgemeinen Struktur A-B werden uretdionhaltige Polyisocyanate zunächst unter Erhalt der Uretdiongruppierung mit Monoalkoholen zu urethanhaltigen, mit Monoaminen und/oder Alkanolaminen und/oder Monohydraziden zu harnstoff- und/oder semicarbazidhaltigen Polymeren umgesetzt, und in einem 2. Schritt werden unter Ringöffung des Uretdion-Ringes mit einer aminfunktionellen Verbindung die erfindungsgemäßen Biuretverbindungen hergestellt.

Uretdione werden, wie dem Fachmann bekannt, durch Addition von monomeren Diisocyanaten unter Verwendung bestimmter Katalysatoren hergestellt (Laas, H.J.; Halpaap, R.; Pedain,J.; J. prakt. Chemie 336 (1994), 185-200). Bevorzugt ist neben dem TDI-Uretdion, erhältlich z. B. als Desmodur® TT/G von der Rhein-Chemie, die Verwendung von HDI-Uretdion, welches kommerziell als Desmodur® N 3400 von BAYER verfügbar ist. Diese Verbindungen stellen Handelsprodukte dar, die häufig nicht in reiner Form vorliegen, sondern die Gemische von Verbindungen ähnlicher Struktur darstellen.

Bei den für Verbindung A eingesetzten Monoalkoholen handelt es sich um aliphatische, cycloaliphatische und araliphatische Alkohole. Bei den aliphatischen Alkoholen werden lineare, verzweigte oder cyclische Alkohole der Kettenlänge C₁ - C₂₂ eingesetzt, wie z. B. Methanol, Ethanol, n-Propanol, 2-Propanol, n-Butanol, Isobutanol, *tert*.-Butanol, Hexanol, Oktanol, Decanol, Dodecanol, Oleylalkohol und Stearylalkohol. Glykolmonoether wie z. B. Ethylenglykol-monopropylether, Ethylenglykol-monobutylether, Ethylenglykol-mono(2-ethylhexylether) oder Ethylenglykol-monophenylether sind ebenfalls miteingeschlossen. Cycloaliphatische Alkohole umfassen z. B. Cyclopentanol und Cyclohexanol. Araliphatische Alkohole wie z. B. Benzylalkohol finden ebenfalls Verwendung. Als Vertreter der cyclischen Alkohole sei z. B. 1-(2-Hydroxyethyl)imidazolidin-2-on genannt. Polymere Alkohole wie Polyolefinmonoole, Polyacrylatmonoole, Polycarbonatmonoole, Polycaprolactonmonoole, Polyoxazolinmonoole oder Polysiloxanmonoole fallen ebenso unter die Erfindung wie Fettalkoholalkoxylate mit variablem Alkoxylierungsgrad, wie sie dem Fachmann unter dem Handelsnamen Lutensol der BASF bekannt sind. Bevorzugt werden Polyoxyalkylenmonoalkohole verwendet, welche Ethylenoxid- und/oder Propylenoxid- und/oder Butylenoxidgruppen enthalten und gegebenenfalls mit Styroloxid modifiziert sind. Besonders bevorzugt ist die Verwendung von Polyoxyalkylenmonoalkoholen wie z. B. MPEG 350, MPEG 500 und MPEG 750, bei denen es sich um Methanol gestartete Polyoxyethylene mit einer terminalen OH-Gruppe handelt. Die Monoalkohole können auch in Mischungen eingesetzt werden.

Bei den für die Herstellung der uretdionhaltigen Verbindung A verwendeten Monoaminen handelt es sich um aliphatische, cycloaliphatische und araliphatische Amine. Bei den aliphatischen Aminen werden lineare, verzweigte oder cyclische Amine der Kettenlänge C₂ - C₂₂ eingesetzt, wie z. B. Ethylamin, Propylamin, Isopropylamin, Butylamin, sek. und tert.-Butylamin, 3-Methyl-1-butanamin, Hexylamin, 2-Ethylhexylamin, Octylamin, Cyclopentylamin, Cyclohexylamin, Tridecylamin, Oleylamin, Octadecylamin und die unter dem Handelsnamen Armeen von Akzo Nobel bekannten Mischungen von C₁₂ - C₂₂ Aminen. Erfindungsgemäß sind neben Polyolefinaminen wie z. B. Polyisobutylenamin auch bevorzugt Polyoxyalkylenmonoamine, welche Ethylenoxid- und/oder Propylenoxidgruppen enthalten und welche unter den Handelsnamen Jeffamine® M 600, M 1000, M 2005 und M 2070 von Huntsman bekannt sind. Bei den araliphatischen Aminen handelt es sich um Produkte, wie z. B. Benzylamin und Furfurylamin. Es können aber auch Hydrazide wie z. B. Benzoesäurehydrazid eingesetzt werden. Die Monoamine können auch als Mischungen eingesetzt werden, ebenso können die Monoamine mit den Monoalkoholen und den Monohydraziden in jedem Verhältnis gemischt, eingesetzt werden.

Ebenfalls können für die Herstellung der uretdionhaltigen Verbindung A Alkanolamine verwendet werden. Als Beispiele seien hier 2-Aminoethanol, 3-Amino-1-propanol, 1-Amino-2-propanol, 2-(2-Aminoethoxy)ethanol, Diethanolamin, 3-(2-Hydroxyethylamino)-1-propanol, Düsopropanolamin und an den OH-Gruppen alkoxylierte Verbindungen davon genannt.

Die Reaktion zwischen dem uretdionhaltigen Polyisocyanat und dem Monoalkohol wird bei Temperaturen zwischen 15 und 90°C, bevorzugt zwischen 20 und 75°C, gegebenenfalls unter Zuhilfenahme eines Katalysators, wie Dibutylzinndilaurat (DBTL), durchgeführt. Die Reaktion zwischen dem uretdionhaltigen Polyisocyanat und dem Monoamin, und/oder dem Alkanolamin und/oder dem Monohydrazid wird bei Temperaturen zwischen 15 und 45°C, bevorzugt zwischen 20 und 30 °C durchgeführt. Dabei ist die Reihenfolge der Zugabe der Co-Reaktanden im allgemeinen beliebig, das uretdionhaltige Polyisocyanat wird vorgelegt, gegebenenfalls in einem inerten Lösemittel, und der Monoalkohol, das Monoamin, das Alkanolamin oder das Monohydrazid werden zugetropft. Ebenso kann der Monoalkohol, das Monoamin, das Alkanolamin oder das Monohydrazid vorgelegt werden, und das uretdionhaltige Polyisocyanat wird zugetropft. Enthält Komponente A sowohl Urethan- als auch Harnstoff- und/oder Semicarbazidgrupen, so wird das uretdionhaltige Polyisocyanat zuerst mit dem Alkohol und danach mit dem Amin und/oder dem Monohydrazid umgesetzt. Gegebenenfalls kann die Reaktion auch in einem inerten Lösemittel durchgeführt werden, wie z. B. Methyoxypropylacetat, Cyclohexan, Toluol, Xylol oder einem höhersiedenden Aromaten wie z. B. Shellsol A.

Die monoaminfunktionellen Verbindungen der allgemeinen Formel (B)

(B) H₂N-R -[Z-R -Z-RJₐ-Z-R

werden unter Bedingungen hergestellt, wie sie dem Fachmann bekannt sind und sind beispielsweise dadurch erhältlich, dass man eine Mischung aus Mono- und Polycarbonsäuren, bevorzugt Dicarbonsäuren, und/oder Dicarbonsäureanhydriden, mit Diaminen, vorzugsweise bei Temperaturen von 100 bis 250 °C, besonders bevorzugt 140 bis 200 °C unter Wasserabscheidung umsetzt. Das Verhältnis Diamin zu Polycarbonsäure zu Monocarbonsäure beträgt 3 zu 2 zu 1 bis 20 zu 19 zu 1, besonders bevorzugt 3 zu 2 zu 1 bis 8 zu 7 zu 1. In einer bevorzugten Ausführungsform lassen sich Verbindungen der allgemeinen Formel (B) dadurch herstellen, dass zuerst die Dicarbonsäure und/oder das Dicarbonsäureanhydrid mit dem Diamin bei Temperaturen von 100 bis 250 °C, besonders bevorzugt 140 bis 200 °C unter Wasserabscheidung zu einem Kondensationsprodukt der allgemeinen Formel (C)

(C) H₂N-R³-[Z-R⁴-Z-R⁵]ₐ-NH₂

mit einer Aminzahl von bevorzugt 5 bis 180 und besonders bevorzugt von 15 bis 100, bezogen auf 100 % aktive Substanz, umgesetzt wird und anschließend bei Temperaturen von 100 bis 250 °C, besonders bevorzugt 140 bis 200 °C, Verbindung (C) mit der Monocarbonsäure unter Wasserabscheidung zu Verbindung (B) umgesetzt wird, wobei dieses Kondensationsprodukt eine Aminzahl von bevorzugt 3 bis 80 und besonders bevorzugt von 8 bis 50, bezogen auf 100 % aktive Substanz, aufweist.

Bei den Diaminen handelt es sich vorzugsweise um aliphatische, aromatische und araliphatische primäre Diamine, wie z. B. Ethylendiamin, Neopentandiamin, 1,2- und 1,3-Propandiamin, 1,4-Butandiamin, 1,5-Pentandiamin, 2-Butyl-2-ethyl-1,5-Pentandiamin, 1,6-Hexamethylendiamin (auch als Lösung in Wasser), 1,8-Octamethylendiamin, 1,12-Dodecamethylendiamin, Cyclohexyldiamin, 4,4'-Diaminodicyclohexylmethan, 3,3'-Dimethyl-4,4'-Diaminodicyclohexylmethan, Isophorondiamin, 4,7-Dioxadecan-1,10-diamin, 4,11-Dioxatetradecan-1,14-diamin, 4,7,10-Trioxadecan-1,13-diamin, Polyoxyalkylen-diamine, welche Ethylenoxid- und/oder Propylenoxid-Gruppen, statistisch oder blockweise angeordnet, enthalten, ein zahlenmittleres Molekulargewicht von 148 bis 4000 g/mol besitzen und beispielsweise als Jeffamine® D 230, D 400, D 2000, D 4000 und Jeffamine® ED 600, ED 900, ED 2003 und EDR 148 von Huntsman erhältlich sind, Polytetrahydrofurandiamine wie z. B. Bis(3-Aminopropyl)polytetrahydrofuran 350, 750, 1100 und 2100 (die Zahlen geben das ungefähre Molekulargewicht an), 4,4'-Diaminodiphenylmethan, 3,3'-Diaminodiphenylsulfon sowie para- und meta-Xylylendiamin. Bevorzugt werden 1,6-Hexamethylendiamin, 1,8-Octamethylendiamin und meta-Xylylendiamin eingesetzt. Ebenfalls können Amine vom Typus H₂N-R-NR-R-NH₂ verwendet werden, wobei R unabhängig für (C₁-C₁₈)-Alkyl oder (C₁-C₄)-Alkoxy steht. Ein Beispiel hierfür ist N,N'-Bis-(3-aminopropyl)methylamin. Es können aber auch Dihydrazide wie z. B. Oxalsäuredihydrazid, Bernsteinsäuredihydrazid oder Adipinsäuredihydrazid eingesetzt werden. Der Einsatz von Mischungen der Diamine, auch mit den Dihydraziden, ist ebenfalls möglich. Die Diamine können auch als Carbonatverbindungen eingesetzt werden, die bei der Kondensationsreaktion mit den Polycarbonsäuren unter Wasserabscheidung und CO₂-Abspaltung zu den erfindungsgemäß bevorzugten Amidgruppierungen reagieren.

Bei den Polycarbonsäuren handelt es sich vorzugsweise um aliphatische, cycloaliphatische oder aromatische, lineare oder verzweigte, gesättigte oder ungesättigte Dicarbonsäuren mit wenigstens 2, besonders bevorzugt 3 bis 40 C-Atomen. Beispiele solcher Polycarbonsäuren sind Adipinsäure, Oxalsäure, Malonsäure, Bernsteinsäure, Glutarsäure, Pimelinsäure, Korksäure, Sebacinsäure, Azelainsäure, Undecandisäure, 1,11-Undecandicarbonsäure, Dodecandisäure, Hexadecandisäure, Dokosandisäure Maleinsäure, Fumarsäure, Terephthalsäure oder Isophthalsäure, alleine oder in Mischungen eingesetzt. Säureanhydride wie Maleinsäureanhydrid, Glutarsäureanhydrid, Phthalsäureanhydrid und Bernsteinsäureanhydrid, welche gegebenenfalls mit Alkyl- oder Alkylengruppen modifiziert sind wie z. B. Dodecenylbernsteinsäureanhydrid, sind ebenfalls in der Erfindung eingeschlossen. Polymere Polycarbonsäuren wie z. B. die Dicarbonsäure des Polybutadiens können ebenso verwendet werden wie hydroxyfunktionelle Polycarbonsäuren wie z. B. Weinsäure, Zitronensäure und Hydroxyphthalsäure. Oxydicarbonsäuren wie 3,6,9-Trioxyundecandisäure und Polyglykoldisäure sind ebenfalls mit eingeschlossen. Dimerisierte Fettsäuren, dem Fachmann als Dimersäuren bekannt, mit einer Kohlenstofflänge von 36 C-Atomen sind ganz besonders bevorzugt. Diese Dimersäuren können sowohl einen geringen Monomergehalt (üblicherweise < 8 Gewichtsprozent), als auch einen Anteil von nicht mehr als 25 Gewichtsprozent an Trimersäure aufweisen.

Bei den Monocarbonsäuren handelt es sich um gesättigte, einfach bis mehrfach ungesättigte, lineare und verzweigte aliphatische Carbonsäuren wie z. B. Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure, Melissinsäure, Lauroleinsäure, Myristoleinsäure, Palmitoleinsäure, Petroselinsäure, Ölsäure, Elaidinsäure, Vaccensäure, Erucasäure, Linolsäure, Linolensäure, Arachidonsäure, Clupanodonsäure, Ricinensäure, α-Elaeostearinsäure, α-Parinarsäure, Kokosölfettsäure, Palmkernölfettsäure, Kokos/Palmkernölfettsäure, Palmölfettsäure, Cottonölfettsäure, Erdnussölfettsäure, Sojaölfettsäure, Sonnenblumenölfettsäure, Rapsölfettsäure und Talgfettsäure. Ebenso finden Ketocarbonsäuren wie beispielsweise Licansäure, als auch aromatische Monocarbonsäuren wie beispielsweise Benzoesäure Verwendung. Als Vertreter der Hydroxycarbonsäuren seien zum Beispiel Glykolsäure, 5-Hydroxyvaleriansäure, 6-Hydroxycapronsäure, Ricinolfettsäure, 12-Hydroxystearinsäure, 12-Hydroxydodecansäure, 5-Hydroxydodecansäure, 5-Hydroxydecansäure oder 4-Hydroxydecansäure genannt. Auch Mischungen der Monocarbonsäuren können eingesetzt werden.

Die Mono- bzw. Polycarbonsäuren können teilweise durch Mono- bzw. Diisocyanate und die Diamine teilweise durch Diole ersetzt werden, wobei dann Ester-, Urethanund/oder Harnstoffgruppen neben den bevorzugten Amidgruppierungen in den Verbindungen der allgemeinen Formeln (B) und (C) vorliegen können.

Bei den Diolen handelt es sich, alleine oder in Mischungen, vorzugsweise um Polyoxyalkylendiole, Polyoxyalkenyldiole, die gegebenenfalls mit (C₁-C₄)-Alkylund/oder -Alkoxygruppen modifiziert sind, um Polyesterdiole, gemischte Polyesterpolyoxyalkylendiole, Polylactondiole, gemischte Polyoxyalkylenpolylactondiole, Polycarbonatdiole, Polyolefindiole, Polyacrylatdiole, alkoxylierte Bisphenol A Diole, um Diole vom Typ α-ω-Dihydroxyalkylensiloxane als auch deren alkoxylierte Verbindungen mit einem mittleren Molekulargewicht Mₙ von 250 bis 5000 g/mol.

Als Diisocyanate können vorzugsweise aliphatische, cycloaliphatische und aromatische Diisocyanate, oder deren Mischungen eingesetzt werden. Beispiele solcher Diisocyanate sind 1,4-Tetramethylendiisocyanat, 1,6-Hexamethylendiisocyanat, 2,2,4-Trimethyl-1,6-Hexamethylendüsocyanat, 1,10-Decamethylendiisocyanat, 1,4-Cyclohexylendüsocyanat, p-Phenylen-DÜsocyanat, m-PhenylenDiisocyanat, 2,6-Toluylendiisocyanat, 2,4-Toluylendiisocyanat und deren Mischungen, p- und m-Xylylendüsocyanat, 1,5-Naphthylendiisocyanat, Isophorondiisocyanat, 4-4' ,-Diisocyanatodicyclohexylmethan, 3,3'-Dimethyl-4,4'-Bisphenylendiisocyanat, 3,3'-Dimethyl-Diisocyanatodiphenylmethan, die Isomerenmischungen 2,4'- und 4-4'-Diisocyanatodiphenylmethan und C₃₆-Dimerdiisocyanat.

Zur Herstellung der erfindungsgemäßen Biuretverbindungen der allgemeinen Struktur A-B werden die Uretdione der allgemeinen Formel A und die monoaminfunktionellen Verbindungen der allgemeinen Formel B bei einer Reaktionstemperatur zwischen 60 und 120 °C, besonders bevorzugt zwischen 75 und 90°C zur Reaktion gebracht. Dabei wird das Verhältnis der Komponenten A und B so gewählt, dass auf 1 mol Verbindung A zwischen 0,8 mol und 1,2 mol, bevorzugt zwischen 0,9 mol und 1,1 mol, besonders bevorzugt 1 mol Verbindung B eingesetzt wird. Die Reaktion kann mit oder ohne Lösemittel durchgeführt werden. Als Lösemittel geeignet sind alle aliphatischen, aromatischen, protischen und aprotischen Lösemittel wie z. B. Methyoxypropylacetat, Cyclohexan, Toluol, Xylol oder höhersiedende Lösemittel wie z. B. Shellsol A oder Exxsol D 40. N-Methylpyrrolidon oder N-Ethylpyrrolidon, aber auch Alkohole wie Ethanol, Propanol, i-Butanol oder Butylglykol sind ebenfalls geeignet. Ebenso kann die Addition in einem Polyether wie z. B. Polypropylenglykol 600 (die Zahl gibt das ungefähre Molekulargewicht an) als Lösemittel durchgeführt werden. Auch Mischungen von Lösemitteln lassen sich verwenden.

Des Weiteren betrifft die vorliegende Erfindung die Verwendung der erfindungsgemäßen Biuretverbindungen der allgemeinen Struktur A-B beziehungsweise der durch das erfindungsgemäße Verfahren erhaltenen Biuretverbindungen der allgemeinen Struktur A-B als Rheologiesteuerungsadditive, insbesondere in lösungsmittelhaltigen und lösungsmittelfreien Lacken auf der Basis von Bindemitteln wie z. B. Polyurethanen (1 K und 2K), Polyacrylaten, Polyester-, Alkyd- und Epoxidharzen, PVC-Plastisolen und -Organosolen, Beschichtungen auf Epoxidbasis und ungesättigten Polyesterharzen. Unter Lacken können auch Nagellacke, wie sie beispielsweise in der Patentschrift US 6,156,325 beschrieben sind, verstanden werden.

Die Einsatzmenge der erfindungsgemäßen Biuretverbindungen der allgemeinen Struktur A-B beträgt 0,05 bis 5,0 Gew.-% Wirksubstanz, bevorzugt 0,1 bis 3,0 Gew.-% Wirksubstanz und besonders bevorzugt 0,2 bis 2,0 Gew.-% Wirksubstanz, bezogen auf das Gewicht der Gesamtformulierung.

Weiterer Gegenstand der Erfindung sind gehärtete und ungehärtete Polymer-Zusammensetzungen enthaltend ein oder mehrere der erfindungsgemäßen Biuretverbindungen der allgemeinen Struktur A-B beziehungsweise der durch das erfindungsgemäße Verfahren erhaltenen Biuretverbindungen der allgemeinen Struktur A-B.

Im folgenden wird die Erfindung anhand von Ausführungsbeispielen näher erläutert.

### Herstellung der erfindungsgemäßen Komponente A:

### Beispiel 1:

In einem 1-Liter-3-Halskolben mit Rührer, Rückflusskühler und Thermometer werden bei Raumtemperatur 195,0 g (0,5 mol) Hexamethylendüsocyanat-Uretdion (Desmodur® N3400 der Fa. Bayer, NCO-Gehalt = 21,5%) und 138,2 g (1,0 mol) Ethylenglykol-monophenylether eingefüllt und auf 70°C erwärmt. Die Reaktion wird solange geführt, bis der NCO-Gehalt kleiner 0,1 % ist. Dann wird auf 50°C abgekühlt.

**Tabelle 1:**

| **Komponenten A:** | | |
|---|---|---|
| Beispiel | Uretdion | Amin-/ Alkoholkomponenten |
| 2 | Hexamethylendiisocyanat-Uretdion | Decanol |
| 3 | Hexamethylendiisocyanat-Uretdion | Oleylalkohol |
| 4 | Hexamethylendiisocyanat-Uretdion | Polyglykol M 350 |
| 5 | Hexamethylendiisocyanat-Uretdion | Benzylamin |
| 6 | Hexamethylendiisocyanat-Uretdion | Tridecylamin |
| 7 | Hexamethylendiisocyanat-Uretdion | Jeffamine® M 600 |
| 8 | Hexamethylendiisocyanat-Uretdion | Jeffamine® M 1000 |
| 9 | Hexamethylendiisocyanat-Uretdion | Polyglykol M 500 / Tri-decylamin (Verhältnis 1 : 1) |

| | | |
|---|---|---|
| Legende1: Jeffamine® M 600: Monoaminfunktioneller EO/PO-Polyether (EO:PO = 1:9), Mn - 600 g/mol, von Huntsman Jeffamine® M 1000: Monoaminfunktioneller EO/PO-Polyether (EO:PO = 19:3), Mn - 1000 g/mol, von Huntsman Polyglykol M 350, 500: Methanol gestartete monohydroxyfunktionelle EO-Polyether, Mn - 350 bzw. 500 g/mol von Clariant | | |

### Herstellung der erfindungsgemäßen Komponente B:

### Beispiel 10:

In einem 1-Liter 3-Halskolben mit Rührer, Wasserabscheider und Thermometer werden nacheinander 227,8 g (0,4 mol) Dimersäure Pripol™ 1009 (Dimerisierte Fettsäure, hydriert, Uniqema, max. 1 % Trimersäure), 69,6 g (0,6 mol) Hexamethylendiamin und 152,1 g Exxsol D40 (dearomatisierter Kohlenwasserstoff, ExxonMobil Chemical) eingewogen und langsam auf 170°C erhitzt. Das bei der Reaktion langsam freiwerdende Wasser wird azeotrop über den Wasserabscheider abgetrennt. Das Kondensationsprodukt besitzt eine Aminzahl von 51. Anschließend werden 57,6 g (0,2 mol) Tallölfettsäure (Mischung von Monocarbonsäuren mit einem hohen Gehalt an Öl- und Linolsäure und ca. 2% Harzsäuren (Abietinsäure), Arizona Chemical GmbH) zugegeben und das bei dieser Reaktion freiwerdende Wasser azeotrop über den Wasserabscheider abgetrennt. Dieses Kondensationsprodukt besitzt eine Aminzahl von 22,7. Danach wird die Reaktionsmischung auf 50°C abgekühlt.

**Tabelle 2:**

| **Komponenten B:** | | | | |
|---|---|---|---|---|
| Beispiel | Diamin | Dicarbonsäure | Monocarbonsäure | Molverhältnis |
| | a | b | c | a:b:c |
| 11 | Hexamethylendiamin | Pripol^{TM} 1006 | Tallölfettsäure | 6 : 5 : 1 |
| 12 | Hexamethylendiamin | Pripol^{TM} 1009 | Heptansäure | 7 : 6 : 1 |
| 13 | Hexamethylendiamin | Pripol^{TM} 1013 | 12-Hydroxystearinsäure | 5 : 4 : 1 |
| 14 | Octamethylendiamin | Empol® 1062 | Stearinsäure | 3 : 2 : 1 |
| 15 | m-Xylylendiamin | Pripol^{TM} 1017 | Tallölfettsäure | 6 : 5 : 1 |
| 16 | Jeffamine® ED 600 | Adipinsäure | Stearinsäure | 4 : 3 : 1 |
| 17 | Jeffamine® D 230 | Adipinsäure / Pripol™ 1006 Verhältnis 1 : 2 | Tallölfettsäure | 5 : 4 : 1 |
| 18 | Hexamethylendiamin | Pripol™ 1006 | Essigsäure | 3 : 2 : 1 |
| 19 | Hexamethylendiamin / m-Xylylendiamin (Verhältnis 2 : 3) | Empol® 1012 | Ölsäure | 4 : 3: 1 |

| | | | | |
|---|---|---|---|---|
| Legende 2: Pripol™ 1006: Dimerisierte Fettsäure (max. 4% Trimersäure), hydriert, von Uniqema Pripol™ 1009: Dimerisierte Fettsäure (max. 1% Trimersäure), hydriert, von Uniqema Pripol™ 1013: Dimerisierte Fettsäure (max. 4% Trimersäure), von Uniqema Pripol™ 1017: Dimerisierte Fettsäure (max. 22% Trimersäure), von Uniqema Empol® 1062: Dimerisierte Fettsäure (max. 3,5% Polycarbonsäure), teilhydriert, von Cognis Empol® 1012: Dimerisierte Fettsäure (max. 5% Polycarbonsäure), hydriert, von Cognis Jeffamine® ED 600: Diaminfunktioneller EO/PO-Polyether (EO:PO = 9:3,6), Mn - 600 g/mol, von Huntsman Jeffamine® D 230: Diaminfunktioneller PO-Polyether, Mn - 230 g/mol, von Huntsman | | | | |

### Herstellung der erfindungsgemäßen Biuret-Addukte:

### Beispiel 20:

In einem 1-Liter-3-Halskolben mit Rührer, Rückflusskühler und Thermometer werden bei Raumtemperatur nacheinander 66,6 g (0,1 mol) des Additionsproduktes aus Beispiel 1 und 247,1 g (0,1 mol; Aminzahl = 22,7) des Kondensationsproduktes aus Beispiel 10 eingewogen und auf 85°C erwärmt. Die Reaktionsmischung wird solange gerührt, bis die Aminzahl kleiner 1 ist. Dann wird das Produkt mit i-Butanol auf einen Festkörper von 30% verdünnt.

**Tabelle 3:**

| **Biuret-Addukte:** | | |
|---|---|---|
| Beispiel | Komponente A | Komponente B |
| 21 | Beispiel 2 | Beispiel 19 |
| 22 | Beispiel 4 | Beispiel 11 |
| 23 | Beispiel 7 | Beispiel 15 |
| 24 | Beispiel 8 | Beispiel 13 |
| 25 | Beispiel 9 | Beispiel 14 |

### Anwendungstechnische Ergebnisse:

Weißlackrezeptur: Worléekyd S 365 Weißlack, 64% Bindemittel, 20% Ti02

| | |
|---|---|
| Worléekyd S 365, 70% in K 030 | 34,4 |
| Disperbyk® 110 | 0,6 |
| RKB-2 (TiO₂) | 20,0 |
| BYK 066 | 0,3 |

Dispergierung: 30 min. Dispermat, 50°C, 8500 U./min. 4 cm Teflonscheibe

| | |
|---|---|
| Nuodex Combi APB | 4,5 |
| Borchi Nox M2 | 0,2 |
| K 030 | 10,4 |
| | 100 |
| Legende 3: Worléekyd S 365: Langölalkydharz, lufttrocknend, der Fa. Worlée Disperbyk® 110: Netz- und Dispergieradditiv (Lösung eines Copolymeren mit sauren Gruppen) der Fa. BYK-Chemie RKB-2: Weißpigment (Titandioxid) der BAYER AG BYK 066: Silikonentschäumer der Fa. BYK-Chemie Nuodex Combi APB: Kombinations Trockner von Sasol Servo BV Borchi Nox M2: Hautverhinderungsmittel (Methylethylketoxim) der Fa. Borchers K 030: Gemisch aus paraffinischen, naphthenischen und aromatischen Kohlenwasserstoffen im Bereich C₈ - C₁₁, von Solvadis Deutschland Additivdosierung: 1 Gew.-% Wirksubstanz, bezogen auf das Gewicht der Gesamtzusammensetzung Einarbeitung: Additiv unter Rühren mit dem Dispermat zugeben Zahnscheibe 2,5 cm, 1200 U./min., 2 min. | |

Abprüfung: Prüfung der rheologischen Wirksamkeit in Form der Ablaufgrenze. Dazu werden die additivierten Lacksysteme mit dem Stufenrakel 50 - 500 µm und 550 - 1000 µm auf Kontrastkarten 2801 von BYK Gardner mit einer automatischer Aufziehbank von BYK Gardner (Geschwindigkeit 3 cm/sec) appliziert. Die Kontrastkarten werden vertikal hängend getrocknet. Das Standvermögen wird in µm nass abgelesen. Dieses ist ein Maß für die rheologische Wirksamkeit. Die Ergebnisse sind in Tabelle 4 dargestellt.

**Tabelle 4:**

| Additiv | Ablaufgrenze [µm] |
|---|---|
| Kontrolle (ohne Additiv) | 150 |
| BYK 411 1 | 450 |
| Beispiel 20 | 900 |
| Beispiel 21 | 550 |
| Beispiel 22 | 800 |
| Beispiel 23 | 650 |
| Beispiel 24 | 1000 |
| Beispiel 25 | 750 |

| | |
|---|---|
| Legende 4: BYK 411: Flüssiges Rheologieadditiv der Fa. BYK-Chemie | |

## Patentansprüche

1. Biuretverbindungen der idealisierten allgemeinen Formel 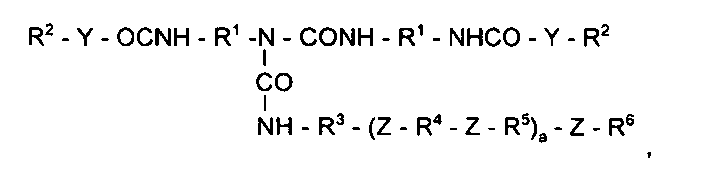 in der
R¹ für einen (C₁-C₂₂)-Alkylen-, (C₃-C₂₂)-Alkenylen-, (C₅-C₁₅)-Cycloalkylen-, Arylen-, (C₇-C₁₂)-Aralkylen-, einen Polyoxyalkylen-Rest oder für einen Polyester-Rest steht,
R² für einen (C₁-C₂₂)-Alkyl-, Hydroxy-(C₁-C₂₂)-Alkyl-, (C₃-C₁₈)-Alkenyl, Aryl-, (C₇-C₁₂)-Aralkyl-, oder (C₅-C₁₂)-Cycloalkyl-Rest, einen Hydroxy-, (C₁-C₂₂)-Alkoxy-, (C₅-C₁₂)-Cycloalkoxy-, oder (C₇-C₁₂)-Aralkoxy-Polyoxyalkylen-Rest, einen (C₁-C₂₂)-Alkanol-, (C₅-C₁₂)-Cycloalkanol-, oder (C₇-C₁₂)-Aralkanol-gestarteten oder einen (C₁-C₂₂)-Alkoxy-, (C₆-C₁₂)-Cycloalkoxy-, oder (C₇-C₁₂)-Aralkoxy-Polyoxyalkylengestarteten Polyester-Rest steht,
Y für gleiche oder verschiedene Reste O, NH, CO-NH-NH oder NH-NH-CO steht R³, R⁴ und R⁵ unabhängig voneinander für einen (C₂-C₄₀)-Alkylen-, (C₃-C₄₀)-Alkenylen-, (C₅-C₄₀)-Cycloalkylen-, Arylen-, (C₇-C₄₀)-Aralkylen- oder Polyoxyalkylen-Rest oder für einen Polyester-Rest stehen,
R⁶ für einen (C₁-C₃₀)-Alkyl-, (C₃-C₂₂)-Alkenyl-, Hydroxyalkyl- und -alkenyl-, (C₄-C₁₃)-Cycloalkyl-, Aryl- oder (C₇-C₁₂)-Aralkyl-Rest steht,
Z für eine oder mehrere der folgenden Gruppen COO, OCO, NHCO, CONH, NHCOO, OOCNH, NHCONH steht und
a für eine Zahl von 1 bis 19 steht.

2. Biuretverbindungen nach Anspruch 1, **dadurch gekennzeichnet, daß** R¹ für einen Hexamethylenrest steht.

3. Biuretverbindungen nach Anspruch 1 oder 2, **dadurch gekennzeichnet, daß** R² für einen (C₁-C₂₂)-Alkyl- oder einen -Alkoxypolyoxyalkylenrest steht.

4. Biuretverbindungen nach Anspruch 1 bis 3, **dadurch gekennzeichnet, daß** die Reste R³ und R⁵ unabhängig voneinander für einen (C₂-C₁₈)-Alkylen-, (C₇-C₁₅)-Aralkylen-Rest, besonders bevorzugt für einen (C₂-C₁₂)-Alkylen-, (C₇-C₁₂)-Aralkylen-Rest, ganz besonders bevorzugt für einen (C₂-C₈)-Alkylen-, (C₇-C₉)-Aralkylen-Rest, wie beispielsweise einen Hexamethylen-, Octamethylen- oder m-Xylylen-Rest stehen.

5. Biuretverbindungen nach Anspruch 1 bis 4, **dadurch gekennzeichnet, daß** R⁴ für einen (C₂-C₄₀)-Alkylen-, (C₃-C₄₀)-Alkenylen-, (C₅-C₄₀)-Cycloalkylen-, Arylen- oder (C₇-C₄₀)-Aralkylen-Rest, bevorzugt für einen (C₃₀-C₄₀)-Alkylen-, (C₃₀-C₄₀)-Alkenylen-, (C₃₀-C₄₀)-Cycloalkylen-, Arylen-, oder (C₃₀-C₄₀)-Aralkylen-Rest steht.

6. Biuretverbindungen nach Anspruch 1 bis 5, **dadurch gekennzeichnet, daß** R⁶ für einen (C₁-C₃₀)-Alkyl- oder einen (C₃-C₂₂)-Alkenyl-Rest steht.

7. Biuretverbindungen nach Anspruch 1 bis 6, **dadurch gekennzeichnet, daß** Z für NHCO oder CONH steht.

8. Biuretverbindungen nach Anspruch 1 bis 7, **dadurch gekennzeichnet, daß** a = 2 bis 7 ist.

9. Verfahren zur Herstellung von Biuretverbindungen nach Anspruch 1 bis 8, **dadurch gekennzeichnet, daß** Uretdione der idealisierten allgemeinen Formel (A) 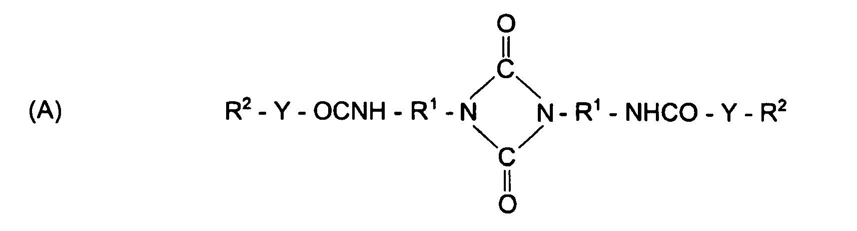 wobei R¹ für einen (C₁-C₂₂)-Alkylen-, (C₃-C₂₂)-Alkenylen-, (C₅-C₁₅)-Cycloalkylen-, Arylen-, (C₇-C₁₂)-Aralkylen-, einen Polyoxyalkylen-Rest oder für einen Polyester-Rest steht,
R² für einen (C₁-C₂₂)-Alkyl-, Hydroxy-(C₁-C₂₂)-Alkyl-, (C₃-C₁₈)-Alkenyl, Aryl-, (C₇-C₁₂)-Aralkyl-, oder (C₅-C₁₂)-Cycloalkyl-Rest, einen Hydroxy-, (C₁-C₂₂)-Alkoxy-, (C₅-C₁₂)-Cycloalkoxy-, oder (C₇-C₁₂)-Aralkoxy-Polyoxyalkylen-Rest, einen (C₁-C₂₂)-Alkanol-, (C₅-C₁₂)-Cycloalkanol-, oder (C₇-C₁₂)-Aralkanol-gestarteten oder einen (C₁-C₂₂)-Alkoxy-, (C₆-C₁₂)-Cycloalkoxy-, oder (C₇-C₁₂)-Aralkoxy-Polyoxyalkylengestarteten Polyester steht,
Y für gleiche oder verschiedene Reste O, NH, CO-NH-NH oder NH-NH-CO steht, mit monoaminfunktionellen Verbindungen der idealisierten allgemeinen Struktur (B)
(B) H₂N - R³ - [Z - R⁴ - Z - R⁵]ₐ - Z - R⁶
umgesetzt werden, wobei R³, R⁴ und R⁵ unabhängig voneinander für einen (C₂-C₄₀)-Alkylen-, (C₃-C₄₀)-Alkenylen-, (C₅-C₄₀)-Cycloalkylen-, Arylen-, (C₇-C₄₀)-Aralkylen- oder Polyoxyalkylen-Rest oder für einen Polyester-Rest stehen,
R⁶ für einen (C₁-C₃₀)-Alkyl-, (C₃-C₂₂)-Alkenyl-, Hydroxyalkyl- und -alkenyl-, (C₄-C₁₃)-Cycloalkyl-, Aryl- oder (C₇-C₁₂)-Aralkyl-Rest steht,
Z für eine oder mehrere der folgenden Gruppen COO, OCO, NHCO, CONH, NHCOO, OOCNH, NHCONH steht und
a für eine Zahl von 1 bis 19 steht.

10. Verwendung von Biuretverbindungen nach Anspruch 1 bis 8 als Mittel zur Rheologiesteuerung.

11. Verwendung von Biuretverbindungen nach Anspruch 1 bis 8 zur Thixotropierung von Beschichtungssystemen, als Antiablaufmittel und/oder Antiabsetzmittel.

## Claims

1. Biuret compounds of the idealized general formula 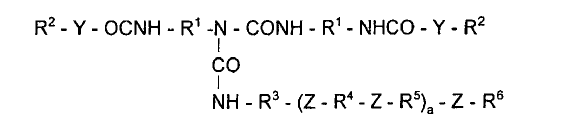 in which
R¹ is a (C₁-C₂₂)-alkylene, (C₃-C₂₂)-alkenylene, (C₅-C₁₅)-cycloalkylene, arylene, (C₇-C₁₂)-aralkylene, a polyoxyalkylene radical or is a polyester radical,
R² is a (C₁-C₂₂)-alkyl, hydroxy-(C₁-C₂₂)-alkyl, (C₃-C₁₈)-alkenyl, aryl, (C₇-C₁₂)-aralkyl, or (C₅-C₁₂)-cycloalkyl radical, a hydroxy-, (C₁-C₂₂)-alkoxy-, (C₅-C₁₂)-cycloalkoxy-, or (C₇-C₁₂)-aralkoxy-polyoxyalkylene radical, or a polyester radical prepared starting from a (C₁-C₂₂)-alkanol, (C₅-C₁₂)-cycloalkanol, or (C₇-C₁₂)-aralkanol or from a (C₁-C₂₂)-alkoxy-, (C₆-C₁₂)-cycloalkoxy-, or (C₇-C₁₂)-aralkoxy-polyoxyalkylene,
Y stands for identical or different radicals O, NH, CO-NH-NH or NH-NH-CO, R³, R⁴ and R⁵ independently of one another are a (C₂-C₄₀)-alkylene, (C₃-C₄₀)-alkenylene, (C₅-C₄₀)-cycloalkylene, arylene, (C₇-C₄₀)-aralkylene or polyoxyalkylene radical or are a polyester radical,
R⁶ is a (C₁-C₃₀)-alkyl, (C₃-C₂₂)-alkenyl, hydroxyalkyl and hydroxyalkenyl, (C₄-C₁₃)-cycloalkyl, aryl or (C₇-C₁₂)-aralkyl radical,
Z stands for one or more of the following groups COO, OCO, NHCO, CONH, NHCOO, OOCNH and NHCONH, and
a is a number from 1 to 19.

2. Biuret compounds according to Claim 1, **characterized in that** R¹ is a hexamethylene radical.

3. Biuret compounds according to Claim 1 or 2, **characterized in that** R² is a (C₁-C₂₂)-alkyl or a -alkoxypolyoxyalkylene radical.

4. Biuret compounds according to Claim 1 to 3, **characterized in that** the radicals R³ and R⁵ independently of one another are a (C₂-C₁₈)-alkylene, (C₇-C₁₅)-aralkylene radical, more preferably a (C₂-C₁₂)-alkylene, (C₇-C₁₂)-aralkylene radical, very preferably a (C₂-C₈)-alkylene, (C₇-C₉)-aralkylene radical, such as a hexamethylene, octamethylene or m-xylylene radical, for example.

5. Biuret compounds according to Claim 1 to 4, **characterized in that** R⁴ is a (C₂-C₄₀)-alkylene, (C₃-C₄₀)-alkenylene, (C₅-C₄₀)-cycloalkylene, arylene or (C₇-C₄₀)-aralkylene radical, preferably a (C₃₀-C₄₀)-alkylene, (C₃₀-C₄₀)-alkenylene, (C₃₀-C₄₀)-cycloalkylene, arylene or (C₃₀-C₄₀)-aralkylene radical.

6. Biuret compounds according to Claim 1 to 5, **characterized in that** R⁶ is a (C₁-C₃₀)-alkyl or a (C₃-C₂₂)-alkenyl radical.

7. Biuret compounds according to Claim 1 to 6, **characterized in that** Z is NHCO or CONH.

8. Biuret compounds according to Claim 1 to 7, **characterized in that** a is = 2 to 7.

9. Process for preparing biuret compounds according to Claim 1 to 8, **characterized in that** uretdiones of the idealized general formula (A) 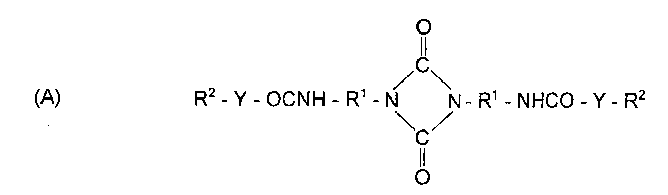 where R¹ is a (C₁-C₂₂)-alkylene, (C₃-C₂₂)-alkenylene, (C₅-C₁₅)-cycloalkylene, arylene, (C₇-C₁₂)-aralkylene, a polyoxyalkylene radical or is a polyester radical,
R² is a (C₁-C₂₂)-alkyl, hydroxy-(C₁-C₂₂)-alkyl, (C₃-C₁₈)-alkenyl, aryl, (C₇-C₁₂)-aralkyl, or (C₅-C₁₂)-cycloalkyl radical, a hydroxy-, (C₁-C₂₂)-alkoxy-, (C₅-C₁₂)-cycloalkoxy-, or (C₇-C₁₂)-aralkoxy-polyoxyalkylene radical, or a polyester prepared starting from a (C₁-C₂₂)-alkanol, (C₅-C₁₂)-cycloalkanol, or (C₇-C₁₂)-aralkanol or from a (C₁-C₂₂)-alkoxy-, (C₆-C₁₂)-cycloalkoxy-, or (C₇-C₁₂)-aralkoxy-polyoxyalkylene,
Y stands for identical or different radicals O, NH, CO-NH-NH or NH-NH-CO,
are reacted with monoamine-functional compounds of the idealized general structure (B)
(B) H₂N-R³-[Z-R⁴-Z-R⁵]ₐ-Z-R⁶
where R³, R⁴ and R⁵ independently of one another are a (C₂-C₄₀)-alkylene, (C₃-C₄₀)-alkenylene, (C₅-C₄₀)-cycloalkylene, arylene, (C₇-C₄₀)-aralkylene or polyoxyalkylene radical or are a polyester radical,
R⁶ is a (C₁-C₃₀)-alkyl, (C₃-C₂₂)-alkenyl, hydroxyalkyl and hydroxyalkenyl, (C₄-C₁₃)-cycloalkyl, aryl or (C₇-C₁₂)-aralkyl radical,
Z stands for one or more of the following groups COO, OCO, NHCO, CONH, NHCOO, OOCNH and NHCONH, and
a is a number from 1 to 19.

10. Use of biuret compounds according to Claim 1 to 8 as rheology control agents.

11. Use of biuret compounds according to Claim 1 to 8 for thixotroping coating systems, as anti-sag agents and/or anti-settling agents.

## Revendications

1. Composés de biuret de formule générale idéalisée 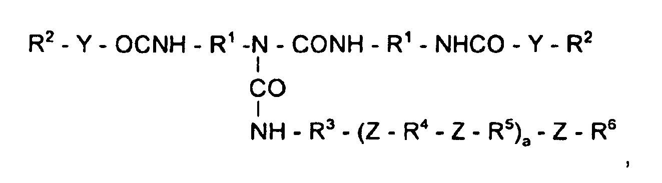 où :
R¹ représente un group fonctionnel (C₁-C₂₂)-alkylène-, (C₃-C₂₂)-alcénylène-, (C₅-C₁₅)-cycloalkylène-, arylène-, (C₇-C₁₂)-aralkylène-, un group fonctionnel polyoxyalkylène ou un group fonctionnel polyester,
R² représente un group fonctionnel (C₁-C₂₂)-alkyle-, hydroxy-(C₁-C₂₂)-alkyle-, (C₃-C₁₈)-alcényle, aryle-, (C₇-C₁₂)-aralkyle-, ou (C₅-C₁₂)-cycloalkyle-, un group fonctionnel hydroxy-, (C₁-C₂₂)-alcoxy-, (C₅-C₁₂)-cycloalcoxy-, ou (C₇-C₁₂)-aralcoxy-polyoxyalkylène, un group fonctionnel polyester initié par (C₁-C₂₂)-alcanole-, (C₅-C₁₂)-cycloalcanole-, ou (C₇-C₁₂)-aralcanole- ou initié par (C₁-C₂₂)-alcoxy-, (C₆-C₁₂)-cyloalcoxy-, ou (C₇-C₁₂)-aralkoxy-polyoxyalkylène,
Y représente des groups fonctionnels identiques ou différents O, NH, CO-NH-NH ou NH-NH-CO,
R³, R⁴ und R⁵ représentent, indépendamment l'un de l'autre, un group fonctionnel (C₂-C₄₀)-alkylène-, (C₃-C₄₀)-alcénylène-, (C₅-C₄₀)-cycloalkylène-, arylène-, (C₇-C₄₀)-aralkylène- ou un group fonctionnel polyoxyalkylène ou un group fonctionnel polyester,
R⁶ représente un group fonctionnel (C₁-C₃₀)-alkyle-, (C₃-C₂₂)-alcényle-, hydroxyalkyle- et -alcényle-, (C₄-C₁₃)-cycloalkyle-, aryle- ou (C₇-C₁₂)-aralkyle-,
Z représente un ou plusieurs des groups suivants: COO, OCO, NHCO, CONH, NHCOO, OOCNH, NHCONH, et
a représente un nombre de 1 à 19.

2. Composés de biuret selon la revendication 1, **caractérisés en ce que** R¹ représente un group fonctionnel hexaméthylène.

3. Composés de biuret selon la revendication 1 ou 2, **caractérisés en ce que** R² représente un group fonctionnel (C₁-C₂₂)-alkyle- ou -alcoxypolyoxy-alkylène.

4. Composés de biuret selon les revendications 1 à 3, **caractérisés en ce que** les groups fonctionnels R³ und R⁵ représentent, indépendamment l'un de l'autre, un group fonctionnel (C₂-C₁₈)-alkylène-, (C₇-C₁₅)-aralkylène-, de préférence un group fonctionnel (C₂-C₁₂)-alkylène-, (C₇-C₁₂)-aralkylène-, et de manière particulièrement préférée un group fonctionnel (C₂-C₈)-alkylène-, (C₇-C₉)-aralkylène-, comme un group fonctionnel hexaméthylène-, octaméthylène- oder m-xylylène-, par exemple.

5. Composés de biuret selon les revendications 1 à 4, **caractérisés en ce que** R⁴ représente un group fonctionnel (C₂-C₄₀)-alkylène-, (C₃-C₄₀)-alcénylène-, (C₅-C₄₀)-cycloalkylène-, arylène- ou (C₇-C₄₀)-aralkylène-, de préférence un group fonctionnel (C₃₀-C₄₀)-alkylène-, (C₃₀-C₄₀)-alcénylène-, (C₃₀-C₄₀)-cycloalkylène-, arylène-, ou (C₃₀-C₄₀)-aralkylène-.

6. Composés de biuret selon les revendications 1 à 5, **caractérisés en ce que** R⁶ représente un groupe fonctionnel (C₁-C₃₀)-alkyle- ou (C₃-C₂₂)-alcényle-.

7. Composés de biuret selon les revendications 1 à 6, **caractérisés en ce que** Z représente NHCO ou CONH.

8. Composés de biuret selon les revendications 1 à 7, **caractérisés en ce que** a = 2 à 7.

9. Procédé de fabrication des composés de biuret selon les revendications 1 à 8, **caractérisé en ce que** des urétdiones de formule générale idéalisée (A) 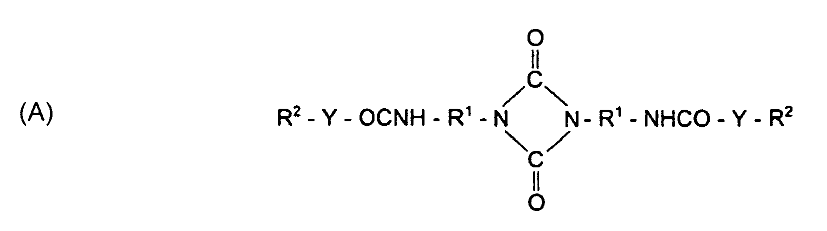 où R¹ représente un group fonctionnel (C₁-C₂₂)-alkylène-, (C₃-C₂₂)-alcénylène-, (C₅-C₁₅)-cycloalkylène-, arylène-, (C₇-C₁₂)-aralkylène-, un group fonctionnel polyoxyalkylène ou un group fonctionnel polyester,
R² représente un group fonctionnel (C₁-C₂₂)-alkyle-, hydroxy-(C₁-C₂₂)-alkyle-, (C₃-C₁₈)-alcényle, aryle-, (C₇-C₁₂)-aralkyle-, ou (C₅-C₁₂)-cycloalkyle-, un group fonctionnel hydroxy-, (C₁-C₂₂)-alcoxy-, (C₅-C₁₂)-cycloalcoxy-, ou (C₇-C₁₂)-aralcoxy-polyoxyalkylène, un group fonctionnel polyester initié par (C₁-C₂₂)-alcanole-, (C₅-C₁₂)-cycloalcanole-, ou (C₇-C₁₂)-aralcanole- ou initié par (C₁-C₂₂)-alcoxy-, (C₆-C₁₂)-cyloalcoxy-, ou (C₇-C₁₂)-aralkoxy-polyoxyalkylène,
Y représente des groups fonctionnels identiques ou différents O, NH, CO-NH-NH ou NH-NH-CO,
sont réagies avec des composés monoamines fonctionnels de la structure générale idéalisée (B)
(B) H₂N - R³ - [Z - R⁴ - Z - R⁵]ₐ- Z - R⁶
ou R³, R⁴ und R⁵ représentent, indépendamment l'un de l'autre, un group fonctionnel (C₂-C₄₀)-alkylène-, (C₃-C₄₀)-alcénylène-, (C₅-C₄₀)-cycloalkylène-, arylène-, (C₇-C₄₀)-aralkylène- ou polyoxyalkylène ou un group fonctionnel polyester,
R⁶ représente un group fonctionnel (C₁-C₃₀)-alkyle-, (C₃-C₂₂)-alcényle-, hydroxyalkyle- et -alcényle-, (C₄-C₁₃)-cycloalkyle-, aryle- ou (C₇-C₁₂)-aralkyle-,
Z représente un ou plusieurs des groups suivants: COO, OCO, NHCO, CONH, NHCOO, OOCNH, NHCONH, et
a représente un nombre de 1 à 19.

10. Utilisation de composés de biuret selon les revendications 1 à 8 en tant qu'agents de contrôle de la rhéologie.

11. Utilisation de composés de biuret selon les revendications 1 à 8 pour la thixotropisation de systèmes de revêtement, en tant qu'agents anti-écoulement et/ou agents de suspension.
